# EUROPEAN PATENT SPECIFICATION

(11) **EP 1 771 135 B1**
(45) Date of publication and mention of the grant of the patent: **10.09.2008**
(21) Application number: 05751017.4
(22) Date of filing: 15.06.2005
(51) Int. Cl.: A61F 13/20

(54) **A HYGIENIC TAMPON AND A METHOD OF MANUFACTURING A HYGIENIC TAMPON**
HYGIENETAMPON UND VERFAHREN ZUR HERSTELLUNG EINES HYGIENETAMPONS
TAMPON HYGIENIQUE ET PROCEDE DE FABRICATION D'UN TAMPON HYGIENIQUE

(30) Priority: 17.06.2004 BR PI0402403
(43) Date of publication of application: 11.04.2007
(73) Proprietor: Johnson & Johnson Industrial Ltda., 12237-350 Sao José dos Campos SP (BR)
(72) Inventor: COSTA, Rogerio, 12608-000 Sáo Paulo, SP (BR)
(74) Representative: Fisher, Adrian John
(86) International application number: PCT/BR2005/000109
(87) International publication number: WO 2005/122986

(56) References cited:
- GB-A- 615 408
- GB-A- 2 064 326
- US-A- 2 188 923
- US-A- 4 212 301

## Description

The present invention relates to a hygienic tampon, particularly used for retaining body exudates such as menstrual flow, as well as to a method of manufacturing a hygienic tampon.

### Description of the Prior Art

Tampons known in the prior art are basically of two basic types based on the structuring/formation thereof.

A first type of tampon comprises an absorbent body made from an absorbent web, as example cotton, rolled up so as to form a cylindrical piece. Before the absorbent web is rolled up, a string may be interlaced at one of the ends of this web, the string remaining interlaced with the web while it is being rolled up. Said string helps the wearer to remove the tampon from the vaginal cavity.

Once the string has been positioned and the web rolled up, the absorbent body undergoes a compacting process that provides this body with the known tampon shapes, maintaining the string fixed and substantially exposed to the absorbent body.

Examples of types of tampons are described hereinafter.

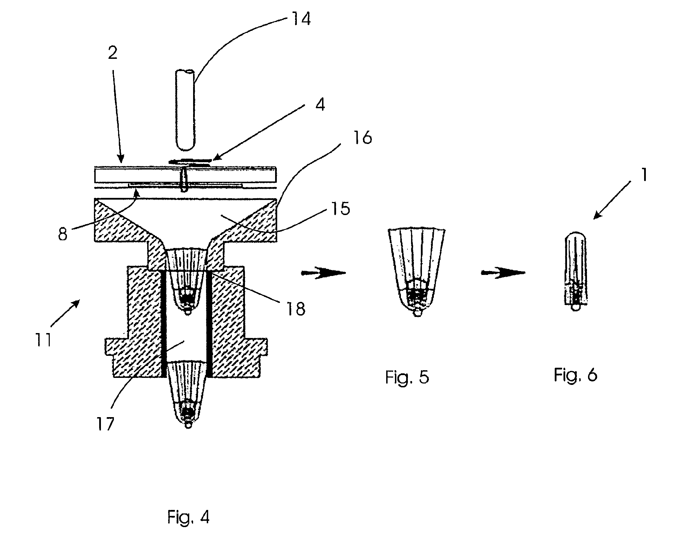

US 6,528,075 describes a tampon comprising a first absorbent element, made from an absorbent material compresses to a substantially cylindrical self-supportable shape, a mass of absorbent material and a string. The string remains substantially exposed, and its portion arranged inside the tampon remains stretched substantially along its length. Said string is fixed to the tampon by known methods, such as seam or treatment with adhesive. The string itself optionally comprises additional absorbent portions/regions, as can be seen in figures 4, 5 and 7 of the document.

US 5,911,712 describes a tampon comprising a core integrally in its central portion, having a small recess in its front out cavity, where the string is embedded, or at least partly embedded, in a spiral way.

US 2,123,750 describes a tampon comprising a substantially cylindrical body portion of absorbent cotton, having an outer portion with a diameter larger than that of said body portion, and a string fixed to said outer portion. Such an arrangement clearly has an extremely uncomfortable general configuration, for both insertion into/removal from the vaginal cavity and mainly during use.

This type of tampon has the great disadvantage that it may get unrolled (a phenomenon called spiraling, that is to say, the various layers of The tampon slide one over the other) and/or the string may get loose from it and might be retained in the vaginal cavity and generate, in extreme cases, the need for medical help to remove it. Thus, this type of tampon lacks safety and comfort to be provided to the wearer.

A second type of tampon comprises an absorbent body made from an absorbent web and a string fixed thereto. The absorbent body is positioned over a device to form the tampon and is punched by applying an axial force perpendicularly onto it, preferably in its central region. In this way, the tampon assumes its conventional shape and has a thin thread of the string hanging free.

As an example of this technique we can recite JP 5-68695, which describes a tampon comprising a laminate composed of a plurality of fibers, containing a string secured to it, which is molded into a cylindrical shape by applying an axial shaping force. The thus obtained tampon; however, has the great drawback that the string may break during a compression step, after application of the axial shaping force, the string becoming substantially exposed.

US 3,863,636 describes a tampon having a cavity with a diameter considerably smaller than the outer diameter of the tampon for arrangement of a string. The string may be arranged inside the tampon with the aid of a vacuum, to provide stretching (which is fundamental to it correct positioning), and subsequent application of a force by means of a piston for inserting the folded string into the cavity. Alternatively, the string may be folded more than once, also with the aid of a piston, which pushes it against a mold provided with cavities and recesses for generating several folds and for subsequent adequate arrangement inside the cavity.

Even though the string virtually remains embedded in the tampon altogether the product disclosed in this document has a number of disadvantages, namely:
- the manufacturing process needs molds/matrixes for the steps of stretching and folding/shaping the string in the tampon cavity;
- the risk of inserting the string into the core inadvertently, which demands at least reprocessing of the unit, or even interruption of the manufacturing operation; and
- the manufacturing process is complex and expensive, because it needs steps to make the cavity, synchronized use of machines, devices and molds for carrying out the steps of stretching, shaping and arranging the string inside the tampon cavity.

In this regard, it remains a need for a tampon provided with a string that provides comfort, total discretion, safety to the wearer and low manufacture cost.

### Objectives of the Invention

A first objective of the present invention is to provide a hygienic tampon that has a substantially non-exposed string and that may be inserted into and removed from the vaginal cavity, providing comfort, total discretion and safety to the wearer and low manufacture cost.

Another objective of the present invention is to provide a method for manufacturing a tampon with the above-mentioned characteristics, which is easy to manufacture and has reduced costs.

### Brief Description of the Invention

The objectives of the invention are achieved by means of a hygienic tampon, particularly for use in a woman's vaginal cavity, comprising an absorbent body and at least one string, the absorbent body being provided with at least one absorbent layer and defining a first inner surface and a second opposed outer surface, the second outer surface being, in use, in contact with the wearer's body, and with at least one hole arranged substantially in the center for fixing the string, the tampon being shaped by applying substantially punctual force perpendicularly to said absorbent body, the string being positioned substantially on the inner surface of the absorbent body and having two well-defined portions,
a first non-exposed portion, positioned on or substantially adjacent the inner surface of the absorbent layer, and,
a second opposite exposed portion facing the second outer surface in a form of a loop and in a manner that it can be handled by the wearer of the tampon,
the second exposed portion of the string being able to be pulled as far as desired or necessary by the user, the string sliding through the hole until almost the whole string configures the exposed portion.

Additionally, the objectives of the present invention are achieved by means of a method of manufacturing a hygienic tampon, particularly a hygienic tampon formed from an absorbent body provided with an inner surface, defined in the preceding paragraph and at least a string, comprising the following steps:
(i) shaping the absorbent body;
(ii) providing at least one hole in the absorbent body;
(iii) fixing the string to the absorbent body through the hole;
(iv) winding the free portion of the string onto the inner surface of the absorbent body in a rolled-up/spiraled condition; and
(v) shaping the absorbent body by using the template.

The present invention has the following advantages:
- the tampon is provided with a string that may remains substantially hidden inside the absorbent body during the process of manufacturing it and that may be removed according to the convenience/desire of each wearer, so that the arrangement of the string enables one to adjust the outer portion to the absorbent body, according to the preference of the wearer;
- the risk of breaking the string from the absorbent body either partly or totally in the compacting step is eliminated and, consequently, the risk of the string breaking while the tampon is being removed from the vaginal cavity becomes extremely improbable;
- the process of manufacturing the tampon is simple and has a reduced cost; and
- the tampon affords comfort, total discretion and safety to the wearer, besides an almost absolute guarantee against breakage of the string at the moment of removing it from the vaginal cavity.

### Brief Description of the Drawings

The present invention will now be described in greater detail with reference to an embodiment represented in the drawings. The figures show:
- Figure 1 is a schematic view of the inner surface of the absorbent body of the hygienic tampon of the present invention;
- Figure 2 is a schematic view of the outer surface of the absorbent body of the hygienic tampon of the present invention;
- Figure 3 is a side view of the layers that form the absorbent body of the hygienic tampon of the present invention;
- Figure 4 is a scheme of the process of manufacturing the hygienic tampon of the present invention;
- Figure 5 is a side view of the tampon obtained by the process illustrated in figure 4, prior to its compaction; and
- Figure 6 is a side view of the finished hygienic tampon of the present invention.

### Detailed Description of the Figures

The present invention relates to a hygienic tampon 1, particularly used in a vaginal cavity, for retaining and absorbing body exudates, as for example menstrual flow or any other flows.

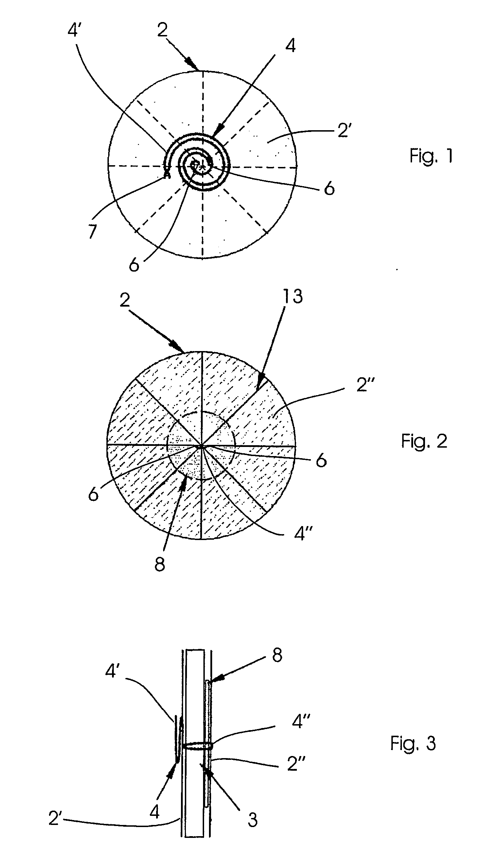

According to a preferred embodiment and as can be seen from figure 1, the hygienic tampon 1 of the present invention is formed by an absorbent body 2 and at least one string 4.

In a first preferred embodiment, the absorbent body 2 is substantially rectangular in shape, whereas in a second preferred embodiment the shape is substantially circular. Evidently, one may provide absorbent bodies 2 of other shapes, as for instance, square, hexagonal, or any other. It should be noted that figures 1 and 2 refer to the second preferred embodiment of the absorbent body 2 and, by inference, of the tampon 1.

The absorbent body 2 is constituted mainly by an absorbent layer 3 composed by an absorbent material, since it accounts for the absorption of the woman's body fluids. Preferably, the absorbent body 2 is constituted by cotton, but it may alternatively be of any other necessary or desirable absorbent material, by preference a substantially hydrophilic material, comprising natural or synthetic fibers, such as peat moss, rayon, and/or else comprising super absorbent elements.

The absorbent body 2 has a first inner surface 2' and a second opposite outer surface 2" facing the outside and that remains in contact with the wearer's vaginal cavity when the tampon 1 is in use.

In a preferred embodiment of the invention and as illustrated in figure 3, the first inner surface 2' corresponds to one of the surfaces of the absorbent layer 3, and the second outer surface 2" corresponds to the other surface of this same absorbent layer 3. Optionally, it is provided at least one retention layer 8 associated to the second outer surface 2".

The retention layer 8 comprises a substantially planar layer of a preferably impermeable polymeric material, such as polyethylene film, or else any other hydrophobic material, as for example a hydrophobic non-woven fabric. The retention layer 8 is substantially smaller in area and thickness if compared with the absorbent layer 3.

The retention layer 8 is preferably fixed to the second outer surface 2" of the absorbent body 2 by means of an adherent layer (not illustrated), as for example, an adhesive tape or a glue, but this may be done by any other way. Alternatively the retention layer 8 may be an integral part of the absorbent body itself.

The main function of the retention layer 8 is to guarantee structural stability of the absorbent body 2 in the region of association of the string 4, preventing it from damaging and/or breaking the absorbent body 2 when the wearer decides to take off the tampon 1 from the vaginal cavity. The fixation of the string 4 will be described in detail later.

Additional, the retention layer 8 has a secondary, but important, function of preventing the occurrence of leaks through the tampon 1, even when the latter is saturated, that is, when it no longer has capacity of absorption, since it functions as an impermeable layer within the tampon itself. Due to the Impermeability of this layer, the fluids accumulated adjacent to it do not pass through it. This property is important, since, when the tampon 1 is being used, the retention layer 8 is adjacent me wearer's undergarment.

By preference, the absorbent body 2 has at least a structural layer, more preferably two structural layers (not illustrated), which serve to impart greater rigidity to the tampon 1. The structural layer(s) may be constituted by a thermoplastic material. Preferably, this thermoplastic material comprises a mesh or a permeable thermoplastic structure comprising thermoplastic polypropylene or polyester fibers coated with polyethylene. However, it is evident that its composition may vary, and one may further conceive an absorbent body 2 without the structural layers.

The absorbent body 2 is provided with at least one, but preferably two through holes 6 arranged substantially in the center, for fixing the string 4. Conveniently, the holes 6 should have their respective diameters substantially equivalent to the diameter of the string 4.

Preferably, the string 4 corresponds to a string or thread, the free ends of which are inserted into the two holes 6 and later tied, forming a knot 7, shown in figure 1. In this way, the string 4 remains fixed to the absorbent body 2 in a loop-shaped way, although it may move axially through the holes 6.

Alternatively, an opened string 4 may be fixed, that is, without its free ends having been tied to each other, as long as one of the free ends has a knot 7, or any other means that prevents it from passing through the hole 8. In this case it is evident that only one of the holes 6 is necessary. One may further provide an open string 4, one end of which is directly fixed to the inner surface 2' of the absorbent body 2.

As can be seen in figures 1 and 2, the string 4 has two well-defined portions. Whatever its configuration, it is associated to the absorbent body 2, so that its two portions are very well delimited, namely: a first non-exposed portion 4', positioned on or substantially adjacent the inner surface of the absorbent layer 2' and a second opposite exposed portion 4" facing the outside and that, therefore, may be handled by the wearer of the tampon 1. It is this second exposed portion that the wearer handles when she wishes to remove the worn tampon 1 from the vaginal cavity.

The first non-exposed portion 4' of the string 4 is shown in detail in figure 1, while the second exposed portion 4" is shown in figure 2.

The string 4 may be manufactured from materials that are conventionally used in tampons, being preferably made from a substantially hydrophobic material.

During the process of manufacturing the tampon 1, which will be explained in detail later, the string 4 is associated to the absorbent body 2, as mentioned above, and passed through the holes 6, so that the exposed portion 4" will configure a mere loop for holding, which enables the wearer to remove the tampon 1 by pulling this string 4. The rest of the string 4 will configure the non-exposed portion 4' and will be positioned on the inner surface of the absorbent layer 2' (therefore, inside the tampon 1).

Thus, the string 4 remains protected against dirt and, very important, it is prevented from being partly or totally broken during the process of manufacturing the tampon 1, when taken out of the package, etc., considerably diminishing the possibility of rupture when the wearer wishes to remove the tampon 1 from the vaginal cavity.

Preferably, the non-exposed portion 4' of the string is positioned on the inner surface 2' of the absorbent layer, in a substantially spiraled way, as can be clearly seen in figures 1, 3 and 4. Thus, there is no possibility of damaging the string 4 during the process of manufacturing the tampon 1, which will be described in detail later.

From the moment when the wearer takes the tampon 1 out of the package, she may handle the loop-shaped non-exposed portion 4' of the string by pulling it as far as desired or necessary. As she performs this task, the string 4 slides through the holes 6, increasing the size of the loop. The wearer may continue pulling the siring 4 until the moment at which the Knot 7, positioned on the inner surface 2' of the absorbent layer, can not pass through the holes 6. In this situation, the positioning of the string 4 is inverted with respect to its initial positioning, that is to say, almost the whole string configures the exposed portion 4", whereas the non-exposed portion 4' then configures a loop. The maximum stretching has been achieved.

It is evident that the wearer may pull the string 4 as she desires, in any proportion, which imparts to the present tampon 1 flexibility of use and potential for satisfying many consumers, increasing its penetration into the consumer market. And, what is very important, without a very high manufacture cost.

In order to manufacture the tampon 1 of the present invention, it is compulsory to use a process of manufacturing a hygienic tampon that is also novel and invective, thus being an object of protection defined in the accompanying claims.

Figure 4 illustrates a scheme of a preferred embodiment of the process of manufacturing the tampon 1, which comprises the following steps:
(i) shaping the absorbent body 2;
(ii) making at least one hole 6 in the absorbent body 2;
(iii) fixing the string 4 to the absorbent body 2 through the hole 6;
(iv) rolling up the free portion 4' of the string 4 on the inner surface 2' of the absorbent body 2 in a rolled-up/spiraled condition;
(v) shaping the absorbent body 2 by means of at least one template 11, by applying an axial force substantially to the center of the absorbent body 2.

The temptate 11 has two regions different from each other. A first region 16, in which the absorbent body 2 is positioned, has a substantially conical cavity 15, the most tapered end having an opening 18 with a calibrated diameter, from which a second region 17 extends in the form of an hollow tube, which will be responsible for determining the final shape of the tampon 1. Preferably, the inner shape of the hollow tube 17 is substantially elliptical; however, it may have other cross-section configurations such as ovoid, cylindrical, octagonal, among others. Further alternatively, the hollow tube 17 may be substantially curved, so as to be similar to a woman's vaginal cavity, for the purpose of obtaining a tampon 1 as anatomic as possible.

Detailing this manufacture process, in step. (i) the absorbent body 2 may be formed from a conventional process of cutting sheets of an absorbent material, using any Known device and/or methods. The absorbent body 2, besides comprising the absorbent layer 3, may further comprise other optional layers, such as the retention layer 8 and structural layers, which may be obtained in a similar way, or else by other known methods. Since the process of manufacturing said layers constituting the tampon 1 is not an object of the invention, it is not described in detail here.

In step (ii) at least one, but preferably two, bores 8 are provides substantial parallel to the central point of the absorbent body 2, for fixing the string 4 (which is done in step (iii)). The way in which the bores 6 are obtained is not an object of this invention; so it may be any method that per forates said absorbent layer 2, retention layer 8 and structural layers (if any), as long as it maintains their integrity in the regions dose to their borders and enables one to maKe the bores 6 with radius substantially equivalent to the diameter of the string 4.

In step (iii), the string 4 is fixed to the absorbent body 2, by association thereof with the bores 6, so that a small exposed end portion 4" of the string 4 will be facing the outside of the absorbent body 2 of the tampon 1. Said exposed end portion 4" has substantial the shape of a loop, to help in inserting and above all removing the tampon 1. Preferably, a knot 7 is provided at the non-exposed end 4'of the string 4.

Optionally, the absorbent body 2 is provided with a plurality of radially arranged creases 13, the function of which being to orient the folding thereof during their compaction.

In step (iv), the non-exposed end 4° of the string is arranged on the second inner surface 2' of the absorbent body 2, preferably in the rolled-up, spiraled condition or the like, allowing the punch 14 to form the absorbent body 2 freely, without the string 4 being directly reached, which might damage it.

In step (v), the absorbent body 2 is positioned in the first fustro-conical shaped region 16 of the template 11, so that the second inner surface 2' will be facing upwards (figure 4).

Additionally, step (v) comprises applying an axial force by means of a punch 14 to the substantially central region of the absorbent body 2, so as to urge it to pass through the hollow tube 17 of the template 11. Since the diameter of the hollow tube 17 is substantially smaller than the diameter of the absorbent body 2, and that the axial force applied by the punch 14 is substantially in its central region, the absorbent body 2 will deform, forming a substantially tubular tampon 1. Details of this step are not given because they are not an object of the present invention.

The punch 14 applies the axial force and commands the passage of the absorbent body 2 through the hollow tube 17.

Preferably, in step (v) the application of an axial force by means of a punch 14 and the passage of the absorbent body 2 through the bore-through tube take place almost simultaneously, but this characteristic is not compulsory.

One may further foresee a variation of the present process, in which a second step of compaction by a pressing equipment (not shown) after step (v) is carried out.

One may also foresee a step of packaging the tampon 1 for better protection and to prevent contamination.

Based on the description of the present application, one can see that the tampon 1 of the present invention has a number of advantages, namely:
- the tampon is provided with a string 4 that may remain substantially hidden inside the absorbant body 2 during the process of manufacturing it and that may be pulled according to the convenience/desire of each wearer, so that the arrangement of the string 4 enables one to adjust the outer portion 4" to the absorbent body 2, according to the preference of the wearer;
- the risk of breaking the string 4 from the absorbent body either partly or totally in the compacting step is substantially reduced and, consequently, the risk of the string 4 breaking while the tampon is being removed from the vaginal cavity becomes extremely improbable;
- the process of manufacturing the tampon 1 is simple and has a reduced cost, being equivalent to that presented by conventional tampons; and
- the tampon 1 affords comfort, total discretion and safety to the wearer, besides an almost absolute guarantee against breakage of the string 4 at the moment of with drawing it from the vaginal cavity.

A preferred embodiment having been described, it should be understood that the scope of the present Invention embraces other possible variations, being limited only by the contents of the accompanying claims, which include the possible equivalents.

## Claims

1. A hygienic tampon (1), particularly for use in a woman's vaginal cavity, comprising an absorbent body (2) and at least one string (4), the absorbent body (2) being provided with at least one absorbent layer (3), defining a first inner surface (2') and a second opposed outer surface (2"), the second outer surface (2") being, in use, in contact with the wearer's body, and also having at least one hole (6) arranged substantially in the center for fixing the string (4), the tampon (1) being shaped, by applying substantially punctual force perpendicularly to said absorbent body (2), the string (4) being positioned substantially on the inner surface (2') of the absorbent body (2) and having two well-defined portions,
a first non-exposed portion (4'), positioned on or substantially adjacent the inner surface (2') of the absorbent layer (3), and,
a second opposite exposed portion (4") facing the second outer surface (2"), in a form of a loop and in a manner that it can be handled by the wearer of the tampon (1),
the tampon (1) being **characterized in that** the second exposed portion (4") of the string (4) is able to be pulled as far as desired or necessary by the user, the string (4) sliding through the hole (6) until almost the whole string configures the exposed portion (4")**.**

2. A tampon according to claim 1, **characterized in that** it comprises two holes in form of through-bores (6) located substantially in the center of the absorbent body (2).

3. A tampon according to any one of the preceding claims, **characterized in that** the string (4) is arranged on the inner surface (2') of the absorbent body in a rolled-up/spiraled condition.

4. A tampon according to any one of claims 1 to 3, **characterized in that** the string (4) has a knot (7).

5. A tampon according to any one of the preceding claims, **characterized in that** the absorbent body (2) comprises at least one impermeable retention layer (8).

6. A tampon according to any one of the preceding claims, **characterized in that** the absorbent body (2) is rectangular in shape.

7. A tampon according to any one of claims 1 to 5, **characterized in that** the absorbent body (2) is circular In shape.

8. A tampon according to any one of the preceding claims, **characterized in that** the absorbent body (2) comprises at least a structural layer, to impart greater rigidity.

9. A tampon according to claim 8, **characterized in that** the absorbent body (2) comprises two structural layers constituted by a thermoplastic material.

10. A tampon according to claim 9, **characterized in that** the thermoplastic material comprises a mesh or a permeable thermoplastic structure comprising thermoplastic polypropylene or polyester fibers coated with polyethylene.

11. A process of manufacturing a hygienic tampon, particularly a hygienic tampon (1) formed from an absorbent body (2) provides with an inner surface (2'), as defined in any one of claims 1 to 10, and at least one string (4), **characterized by** comprising the following steps:
(i) shaping the absorbent body (2);
(ii) making a bore (6) in the absorbent body (2);
(iii) fixing the string (4) to the absorbent body (2) through the bore (6);
(iv) rolling up a non-exposed portion (4') of the string (4) on the inner surface (2') of the absorbent body (2) in a rolled-up/spiraled condition
(v) shaping the absorbent body (2) by means of the template (11).

12. A process according to claim 11, **characterized in that**, in step (iii), the string (4) is fixed to the absorbent body (2) by association with the bores (6), so that a small exposed outer portion (4") of the string (4) will face outwardly to the absorbent body (2) of the tampon (1).

13. A process according to claim 11, **characterized in that** step (v) comprises positioning the absordent body (2) provided with a string (4) at a template (11).

14. A process according to claim 11, **characterized in that** step (v) comprises applying an axially force substantially in the center of the absorbent body (2), by means of a punch (14).

15. A process according to claim 11, **characterized by** additionally comprising a second step of compacting the tampon (1) after step (v).

16. A process according to claim 11, **characterized by** further comprising a step of packaging the tampon (1) after step (v).

## Patentansprüche

1. Hygienetampon (1), insbesondere zur Verwendung in der Vaginalhöhle einer Frau, umfassend einen absorbierenden Körper (2) und mindestens einen Faden (4), wobei der absorbierende Körper (2) mit mindestens einer absorbierenden Schicht (3) versehen ist, eine erste Innenfläche (2') und eine zweite gegenüberliegende Außenfläche (2") bildet, wobei die zweite Außenfläche (2") im Gebrauch mit dem Körper des Trägers in Kontakt steht, und auch mindestens ein Loch (6), das im wesentlichen in der Mitte angeordnet ist, zum Befestigen des Fadens (4) aufweist, wobei der Tampon (1) durch Ausüben von im wesentlichen punktförmiger Kraft senkrecht zum absorbierenden Körper (2) geformt ist, der Faden (4) im wesentlichen auf der Innenfläche (2") des absorbierenden Körpers (2) positioniert ist und zwei klar abgegrenzte Abschnitte aufweist,
einen ersten nicht freiliegenden Abschnitt (4'), der auf oder im wesentlichen benachbart zur Innenfläche (2') der absorbierenden Schicht (3) positioniert ist, und
einen zweiten gegenüberliegenden freiliegenden Abschnitt (4"), der zur zweiten Außenfläche (2") gewandt ist und in Form einer Schlaufe und in einer Weise vorliegt, dass er von dem Träger des Tampons (1) gehandhabt werden kann, wobei der Tampon (1) **dadurch gekennzeichnet ist, dass** der zweite freiliegende Abschnitt (4") des Fadens (4) nach Wunsch oder Bedarf vom Benutzer gezogen werden kann, wobei der Faden (4) durch das Loch (6) gleitet, bis nahezu der gesamte Faden den freiliegenden Abschnitt (4") bildet.

2. Tampon nach Anspruch 1, **dadurch gekennzeichnet, dass** er zwei Löcher in Form vo Durchgangsbohrungen (6) aufweist, die im wesentlichen in der Mitte des absorbierenden Körpers (2) angeordnet sind.

3. Tampon nach einem der vorangehenden Ansprüche, **dadurch gekennzeichnet, dass** der Faden (4) auf der Innenfläche (2') des absorbierenden Körpers in einem eingerollten/spiralförmigen Zustand angeordnet ist.

4. Tampon nach einem der Ansprüche 1 bis 3, **dadurch gekennzeichnet, dass** der Faden (4) einen Knoten (7) aufweist.

5. Tampon nach einem der vorangehenden Ansprüche, **dadurch gekennzeichnet, dass** der absorbierende Körper (2) mindestens eine undurchlässige Rückhalteschicht (8) aufweist.

6. Tampon nach einem der vorangehenden Ansprüche, **dadurch gekennzeichnet, dass** der absorbierende Körper (2) eine rechteckige Gestalt aufweist.

7. Tampon nach einem der Ansprüche 1 bis 5, **dadurch gekennzeichnet, dass** der absorbierende Körper (2) eine kreisförmige Gestalt aufweist.

8. Tampon nach einem der vorangehenden Ansprüche, **dadurch gekennzeichnet, dass** der absorbierende Körper (2) mindestens eine Strukturschicht für größere Steifheit aufweist.

9. Tampon nach Anspruch 8, **dadurch gekennzeichnet, dass** der absorbierende Körper (2) zwei Strukturschichten aufweist, die durch ein thermoplastisches Material gebildet sind.

10. Tampon nach Anspruch 9, **dadurch gekennzeichnet, dass** das thermoplastische Material ein Netz oder eine durchlässige thermoplastische Struktur, umfassend thermoplastisches Polypropylen oder Polyesterfasern, die mit Polyethylen beschichtet sind, aufweist.

11. Verfahren zur Herstellung eines Hygienetampons, insbesondere eines Hygienetampons (1), der aus einem absorbierenden Körper (2) gebildet ist, der mit einer Innenfläche (2') versehen ist, nach einem der Ansprüche 1 bis 10, und mindestens einem Faden (4), **gekennzeichnet durch** die folgenden Schritte:
(i) Formen des absorbierenden Körpers (2);
(ii) Ausbilden einer Bohrung (6) in dem absorbierenden Körper (2);
(iii) Befestigen des Fadens (4) an dem absorbierenden Körper (2) **durch** die Bohrung (6);
(iv) Einrollen eines nicht freiliegenden Abschnittes (4') des Fadens (4) auf der Innenfläche (2') des absorbierenden Körpers (2) in einem eingerollten/spiralförmigen Zustand,
(v) Formen des absorbierenden Körpers (2) mittels der Schablone (11).

12. Verfahren nach Anspruch 11, **dadurch gekennzeichnet, dass** in Schritt (iii) der Faden (4) an dem absorbierenden Körper (2) durch Verbinden mit den Bohrungen (6) befestigt wird, so dass ein geringer freiliegender Körper äußerer Abschnitt (4") des Fadens (4) nach außen zum absorbierenden Körper (2) des Tampons (1) gewandt sein wird.

13. Verfahren nach Anspruch 11, **dadurch gekennzeichnet, dass** Schritt (v) Positionieren des absorbierenden Körpers (2), der mit einem Faden (4) versehen ist, auf der Schablone (11) aufweist.

14. Verfahren nach Anspruch 11, **dadurch gekennzeichnet, dass** Schritt (v) Ausüben einer axialen Kraft im wesentlichen in der Mitte des absorbierenden Körpers (2) mittels eines Stempels (14) aufweist.

15. Verfahren nach Anspruch 11, **dadurch gekennzeichnet, dass** es zusätzlich einen zweiten Schritt des Kompaktierens des Tampons (1) nach Schritt (v) aufweist.

16. Verfahren nach Anspruch 11, **dadurch gekennzeichnet, dass** es ferner einen Schritt des Verpackens des Tampons (1) nach Schritt (v) aufweist.

## Revendications

1. Tampon hygiénique (1), destiné à être utilisé en particulier dans la cavité vaginale d'une femme, comprenant un corps absorbant (2) et au moins une ficelle (4), le corps absorbant (2) comportant au moins une couche absorbante (3), définissant une première surface interne (2') et une seconde surface externe opposée (2"), la seconde surface externe (2") étant utilisée au contact du corps de l'utilisatrice, et ayant au moins un alésage (6) disposé sensiblement au centre pour fixer la ficelle (4), le tampon (1) étant formé en exerçant une force sensiblement ponctuelle perpendiculairement audit corps absorbant (2), la ficelle (4) étant positionnée sensiblement sur la surface interne (2') du corps absorbant (2) et ayant deux parties bien définies,
une première partie non exposée (4'), positionnée sur, ou sensiblement adjacente à, la surface interne (2') de la couche absorbante (3) et
une seconde partie exposée opposée (4"), faisant face à la seconde surface externe (2") sous la forme d'une boucle de façon à pouvoir être saisie par l'utilisatrice du tampon (1),
le tampon (1) étant **caractérisé en ce que** la seconde partie exposée (4") de la ficelle (4) peut être tirée aussi loin que souhaité ou nécessaire par l'utilisatrice, la ficelle (4) glissant à travers le trou (6) jusqu'à ce que presque toute la ficelle forme la partie exposée (4").

2. Tampon selon la revendication 1, **caractérisé en ce qu'**il comprend deux trous sous la forme d'alésages traversants (6) situés sensiblement au centre du corps absorbant (2).

3. Tampon selon l'une quelconque des revendications précédentes, **caractérisé en ce que** la ficelle (4) est disposée sur la surface interne (2') du corps absorbant à l'état enroulé/en spirale.

4. Tampon selon l'une quelconque des revendications 1 à 3, **caractérisé en ce que** la ficelle (4) a un noeud (7).

5. Tampon selon l'une quelconque des revendications précédentes, **caractérisé en ce que** le corps absorbant (2) comprend au moins une couche de retenue imperméable (8).

6. Tampon selon l'une quelconque des revendications précédentes, **caractérisé en ce que** le corps absorbant (2) est de forme rectangulaire.

7. Tampon selon l'une quelconque des revendications 1 à 5, **caractérisé en ce que** le corps absorbant (2) est de forme circulaire.

8. Tampon selon l'une quelconque des revendications précédentes, **caractérisé en ce que** le corps absorbant (2) comprend au moins une couche structurelle, pour conférer une plus grande rigidité.

9. Tampon selon la revendication 8, **caractérisé en ce que** le corps absorbant (2) comprend deux couches structurelles constituées d'un matériau thermoplastique.

10. Tampon selon la revendication. 9, **caractérisé en ce que** le matériau thermoplastique est constitué de mailles ou d'une structure thermoplastique perméable constituée de fibres thermoplastiques de polypropylène ou de polyester revêtues de polyéthylène.

11. Procédé de fabrication d'un tampon hygiénique, en particulier un tampon hygiénique (1) formé d'un corps absorbant (2) comportant une surface interne (2'), telle que définie dans l'une quelconque des revendications 1 à 10, et au moins une ficelle (4), **caractérisé en ce qu'**il comprend les étapes consistant à :
(i) former le corps absorbant (2) ;
(ii) creuser un alésage (6) dans le corps absorbant (2) ;
(iii) fixer la ficelle (4) au corps absorbant (2) à travers l'alésage (6) ;
(iv) enrouler une partie non exposée (4') de la ficelle (4) sur la surface interne (2') du corps absorbant (2) à l'état enroulé/en spirale ;
(v) former le corps absorbant (2) au moyen du gabarit (11).

12. Procédé selon la revendication 11, **caractérisé en ce que**, à l'étape (iii), la ficelle (4) est fixée au corps absorbant (2) par association avec les alésages (6), de façon à ce qu'une petite partie externe exposée (4'') de la ficelle (4) soit orientée face à l'extérieur sur le corps absorbant (2) du tampon (1).

13. Procédé selon la revendication 11, **caractérisé en ce que** l'étape (v) consiste en la mise en place du corps absorbant (2) muni d'une ficelle (4) dans un gabarit (11).

14. Procédé selon la revendication 11, **caractérisé en ce que** l'étape (v) consiste à exercer une force dans le sens axial sensiblement au centre du corps absorbant (2) au moyen d'un poinçon (14).

15. Procédé selon la revendication 11, **caractérisé en ce qu'**il comprend en outre une seconde étape consistant à comprimer le tampon (1) après l'étape (v).

16. Procédé selon la revendication 11, **caractérisé en ce qu'**il comprend en outre une étape consistant à emballer le tampon (1) après l'étape (v).
